(19) Europäisches Patentamt
European Patent Office
Office européen des brevets

(11) Veröffentlichungsnummer: **0 184 068**
**A1**

# (12) EUROPÄISCHE PATENTANMELDUNG

(21) Anmeldenummer: **85114677.9**

(22) Anmeldetag: **19.11.85**

(51) Int. Cl.⁴: **C 07 C 121/46**
C 07 C 120/00, C 09 K 19/32
G 02 F 1/13

(30) Priorität: **01.12.84 DE 3443929**

(43) Veröffentlichungstag der Anmeldung:
**11.06.86 Patentblatt 86/24**

(84) Benannte Vertragsstaaten:
**CH DE GB LI**

(71) Anmelder: **Merck Patent Gesellschaft mit beschränkter Haftung**
**Frankfurter Strasse 250**
**D-6100 Darmstadt(DE)**

(72) Erfinder: **Hittich, Reinhard, Dr.**
**Am Kirchberg 11**
**D-6101 Modautal 1(DE)**

(72) Erfinder: **Weber, Georg**
**Wilhelm-Leuschner-Strasse 38**
**D-6106 Erzhausen(DE)**

(72) Erfinder: **Sucrow, Wolfgang, Prof.**
**Hüfferweg 13**
**D-4790 Paderborn(DE)**

(72) Erfinder: **Geschwinder, Peter, Dr.**
**Mörickestrasse 22**
**D-4790 Paderborn(DE)**

(54) **Carbonitrile.**

(57) Carbonitreil der Formel I

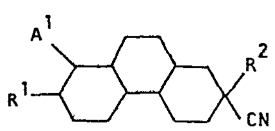

I

worin $R^1$ und $R^2$ jeweils eine Alkylgruppe mit 1 – 12 C-Atomen bedeutet, worin auch eine oder zwei nicht benachbarte $CH_2$-Gruppen durch –O–, –O–CO–, –CO–O– und/oder –CH=CH– ersetzt sein können, und

$A^1$ H, oder 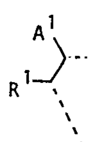 eine Gruppe der Formel 1 bedeutet

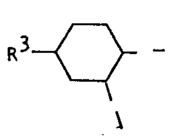

(1)

worin $R^3$ eine der Bedeutungen von $R^1$ hat, können als Komponenten flüssigkristallinder Phasen verwendet werden.

EP 0 184 068 A1

Merck Patent Gesellschaft
mit beschränkter Haftung

6100 Darmstadt

Die Erfindung betrifft neue Carbonitrile der Formel I

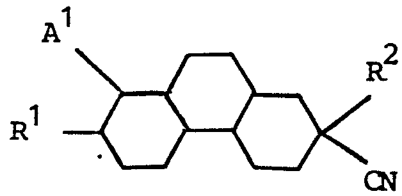

I

worin $R^1$ und $R^2$ jeweils eine Alkylgruppe mit 1 - 12 C-Atomen bedeutet, worin auch eine oder zwei nicht benachbarte $CH_2$-Gruppen durch -O-, -O-CO-, -CO-O- und/oder -CH=CH- ersetzt sein können, und

$A^1$ H, oder 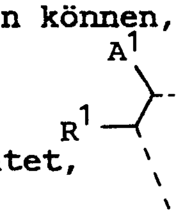 eine Gruppe der Formel 1 bedeutet,

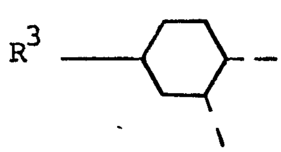

(1)

worin $R^3$ eine der Bedeutungen von $R^1$ hat.

Ähnliche Verbindungen sind z. B. aus der DE-OS 33 19 781 bekannt. Die Verbindungen der Formel I können wie ähnliche Verbindungen als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen mit stark negativer dielektrischer Anisotropie und damit kleiner Schwellen- bzw. Steuerspannung elektrooptischer Effekte, sehr kleiner optischer Anisotropie, vergleichsweise niedriger Viskosität und relativ hohen Klärpunkten herstellbar.

Mit der Bereitstellung der Verbindungen der Formel I wird außerdem ganz allgemein die Palette der flüssigkristallinen Substanzen, die sich unter verschiedenen anwendungstechnischen Gesichtpunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als

Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie oder den Klärpunkt eines solchen Dielektrikums zu beeinflussen. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine Verbindung der Formel I, worin $R^2$ H bedeutet, mit einer Verbindung der Formel II

$$R^2\text{-}X \qquad \text{II}$$

worin X Cl, Br, J, OH oder eine reaktionsfähig veresterte OH-Gruppe bedeutet und
$R^2$ die angegebene Bedeutung hat,

umsetzt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Die Verbindungen der Formel I umfassen dementsprechend Perhydrophenanthrenderivate der Teilformel Ia und Perhydrochrysenderivate der Teilformel Ib:

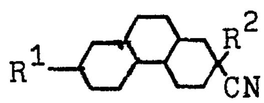

Ia

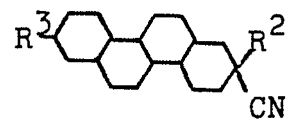

Ib

In den Verbindungen der vor- und nachstehenden Formeln bedeuten $R^1$ und $R^3$ vorzugsweise Alkyl, ferner Alkoxy oder eine andere Oxaalkylgruppe. $R^2$ ist vorzugsweise Alkyl.

X ist vorzugsweise Cl oder Br, aber auch J, OH oder reaktionsfähig verestertes OH wie Alkylsulfonyloxy mit insbesondere 1 - 6 C-Atomen (z. B. Methylsulfonyloxy) oder Arylsulfonyloxy mit insbesondere 6 - 10 C-Atomen (z. B. Phenyl-, p-Tolyl- oder Naphthylsulfonyloxy).

In den Verbindungen der vor- und nachstehenden Formeln können die Alkylreste, in denen auch eine (Alkoxy bzw. Oxaalkyl) oder zwei nicht benachbarte (Alkoxyalkoxy bzw. Dioxyalkyl) $CH_2$-Gruppen durch O-Atome ersetzt sein können, geradkettig,

oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2-(=Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Falls in den Alkylresten der vor- und nachstehenden Formeln eine oder zwei $CH_2$-Gruppen durch -CH=CH- ersetzt sind, sind die trans-Alkylengruppen bevorzugt. Diese Alkylengruppen können somit 1 bis 13 C-Atome (bzw. 14 C-Atome bei Dienylgruppen) enthalten.

Verbindungen der Formel I sowie Ia und Ib mit verzweigten Flügelgruppen $R^1$ , $R^2$ bzw. $R^3$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl,

2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Unter den Verbindungen der Formeln I sowie Ia und Ib sind diejenigen bevorzugt, in denen mindestens einer der darin enthaltenen Reste eine der angegebenen bevorzugten Bedeutungen hat. Besonders bevorzugte kleinere Gruppen vcon Verbindungen sind diejenigen der Formeln I 1 bis I 8,

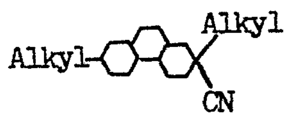

I 1

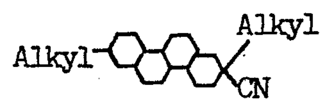

I 5

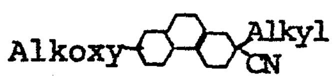

I 2

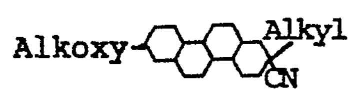

I 6

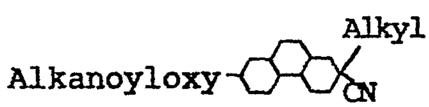

I 3

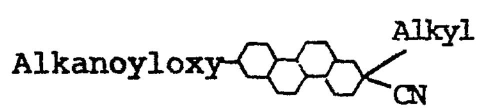

I 7

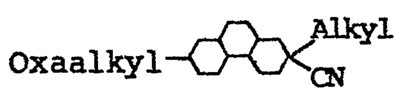

I 4

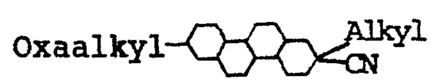

I 8

worin Alkyl, Alkoxy, Alkanoyloxy und Oxaalkyl jeweils unabhängig voneinander geradkettige Gruppen mit 1 - 12, insbesondere 2 - 7 C-Atomen, bedeuten.

Die Verbindungen der Teilformeln Ia und Ib besitzen mehrere Asymmetriezentren. Sie können daher bei ihrer Herstellung als Racemate oder, falls optisch aktive Ausgangsstoffe verwendet werden, auch in optisch aktiver Form erhalten werden. Optisch aktive Enantiomere der Formel I können beispielsweise vorteilhaft als Zusätze für ferroelektrische smektische Phasen verwendet werden. Falls Gemische von Racematen anfallen, können daraus die einzelnen Racemate, beispielsweise durch Umkristallisieren der Racemate selbst oder ihrer diastereomeren Derivate aus inerten Lösungsmitteln, in reiner Form isoliert werden.

Die Synthese wird jedoch vorzugsweise so geführt, daß überwiegend oder ausschließlich die bevorzugten Racemate der Konfigurationen Iaa bzw. Iba gebildet werden:

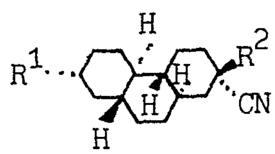

Iaa

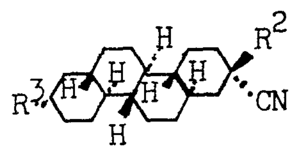

Iba

worin die Substituenten $R^1$ und $R^2$ bzw. $R^3$ und $R^2$ equatorial, die CN-Gruppe jeweils axial steht.

Die Verbindungen der Formel I können nach an sich bekannten Methoden hergestellt werden, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung der Formel I, worin $R^2$ H bedeutet, mit einer Verbindung der Formel II

$R^2$-X II

worin X Cl, Br, J, OH oder eine reaktionsfähig veresterte OH-Gruppe bedeutet und
$R^2$ die angegebene Bedeutung hat,

umsetzt.

Die Herstellung der Perhydrophenanthrencarbonitrile ist aus DE-OS 31 48 448 bekannt; die Perhydrochrysencarbonitrile können nach entsprechenden Verfahren hergestellt werden.

Das Nitril wird zweckmäßig zunächst mit einer starken Base wie NaH, $NaNH_2$, Lithiumdiisopropylamid, -piperidid oder -2,5-diisopropylpiperidid oder K-tert.-Butylat in das entsprechende Carbanion übergeführt, vorzugsweise in einem inerten Lösungsmittel, beispielsweise einem Kohlenwasserstoff wie Toluol, einem Ether wie THF oder Dioxan, einem Amid wie DMF, einem Sulfoxid wie Dimethylsulfoxid oder einem Gemisch derartiger Lösungsmittel. Nach Zugabe von

II (worin X von OH verschieden ist) hält man zweckmäßig 0,5 bis 16 Stunden bei Temperaturen zwischen -30° und 100°. Eine Umsetzung mit II (X = OH) gelingt dagegen zweckmäßig in Gegenwart von Azodicarbonsäureestern/Triphenylphosphin in THF bei Temperaturen zwischen etwa -30° und +30°.

Die erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexyl-ethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

R'-L-G-E-R''    II

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | -COO-Phe-COO- |

oder eine C-C-Einfachbindung, Y Halogen, vorzugsweise Chlor, oder -CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen Phasen enthalten etwa 0,1 bis 99, vorzugsweise 10 bis 95 %, einer oder mehrerer Verbindungen der Formel I.

Weiterhin bevorzugt sind erfindungsgemäße Dielektrika enthaltend 0,1 bis 40, vorzugsweise 0,5 bis 30 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol.Cryst.Liq.Cryst. Band 24, Seiten 249 - 258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Beispiel 1

Man löst 30 g 2ß-Cyan-7α-n-hexyl-4aß-H-4bα-H-8aß-H-10aα-H-perhydrophenanthren (Synthese beschrieben in DE-OS 31 48 448) und 36 g n-Propylbromid in 100 ml Toluol, versetzt mit 4,3 g $NaNH_2$ (50 % in Toluol) und kocht 5 Stunden. Nach üblicher Aufarbeitung erhält man 2α-Cyan-2ß-n-propyl-7α-n-hexyl-4aß-H-4bα-H-8aß-H-10aα-H-perhydrophenanthren.

Analog erhält man durch Alkylierung der entsprechenden Nitrile (im Nachfolgenden wird 4aß-H-4bα-H-8aß-H-10aα-H-perhydrophenanthren kurz als "perhydrophenanthren" bezeichnet):

2α-Cyan-2ß-propyl-7α-ethyl-perhydrophenanthren
2α-Cyan-2ß-propyl-7α-propyl-perhydrophenanthren
2α-Cyan-2ß-propyl-7α-butyl-perhydrophenanthren
2α-Cyan-2ß-propyl-7α-pentyl-perhydrophenanthren
2α-Cyan-2ß-propyl-7α-heptyl-perhydrophenanthren

2α-Cyan-2β-ethyl-7α-ethyl-perhydrophenanthren
2α-Cyan-2β-ethyl-7α-propyl-perhydrophenanthren
2α-Cyan-2β-ethyl-7α-butyl-perhydrophenanthren
2α-Cyan-2β-ethyl-7α-pentyl-perhydrophenanthren
2α-Cyan-2β-ethyl-7α-heptyl-perhydrophenanthren
2α-Cyan-2β-butyl-7α-ethyl-perhydrophenanthren
2α-Cyan-2β-butyl-7α-propyl-perhydrophenanthren
2α-Cyan-2β-butyl-7α-butyl-perhydrophenanthren
2α-Cyan-2β-butyl-7α-pentyl-perhydrophenanthren
2α-Cyan-2β-butyl-7α-heptyl-perhydrophenanthren
2α-Cyan-2β-pentyl-7α-ethyl-perhydrophenanthren
2α-Cyan-2β-pentyl-7α-propyl-perhydrophenanthren
2α-Cyan-2β-pentyl-7α-butyl-perhydrophenanthren
2α-Cyan-2β-pentyl-7α-pentyl-perhydrophenanthren
2α-Cyan-2β-pentyl-7α-heptyl-perhydrophenanthren
2α-Cyan-2β-hexyl-7α-ethyl-perhydrophenanthren
2α-Cyan-2β-hexyl-7α-propyl-perhydrophenanthren
2α-Cyan-2β-hexyl-7α-butyl-perhydrophenanthren
2α-Cyan-2β-hexyl-7α-pentyl-perhydrophenanthren
2α-Cyan-2β-hexyl-7α-heptyl-perhydrophenanthren
2α-Cyan-2β-heptyl-7α-ethyl-perhydrophenanthren
2α-Cyan-2β-heptyl-7α-propyl-perhydrophenanthren
2α-Cyan-2β-heptyl-7α-butyl-perhydrophenanthren
2α-Cyan-2β-heptyl-7α-pentyl-perhydrophenanthren
2α-Cyan-2β-heptyl-7α-heptyl-perhydrophenanthren
2α-Cyan-2β-pentyl-7α-methoxy-perhydrophenanthren
2α-Cyan-2β-pentyl-7α-ethoxy-perhydrophenanthren
2α-Cyan-2β-pentyl-7α-propoxy-perhydrophenanthren
2α-Cyan-2β-pentyl-7α-butoxy-perhydrophenanthren
2α-Cyan-2β-pentyl-7α-propionyloxy-perhydrophenanthren
2α-Cyan-2β-pentyl-7α-butyryloxy-perhydrophenanthren
2α-Cyan-2β-pentyl-7α-pentanoyloxy-perhydrophenanthren
2α-Cyan-2β-pentyl-7α-2-oxabutyl-perhydrophenanthren
2α-Cyan-2β-pentyl-7α-3-oxabutyl-perhydrophenanthren

Beispiel 2

Man löst 3,4 g 2ß-Cyan-8α-n-pentyl-4aß-H-4bα-H-10aα-H-10bß-H-12aα-H-perhydrochrysen (Synthese beschrieben in der deutschen Patentanmeldung P 34 26 039) and 3,6 g n-Propylbromid in 15 ml Toluol, versetzt mit 0,4 g $NaNH_2$ (50 % in Toluol) und kocht 6 Stunden. Nach üblicher Aufarbeitung erhält man 2α-Cyan-2ß-n-propyl-8α-n-pentyl-4aß-H-4bα-H-10aα-H-10bß-H-12aα-H-perhydrochrysen.

Analog erhält man durch Alkylierung der entsprechenden Nitrile (im Nachfolgenden wird 4aß-H-4bα-H-10aα-H-10bß-H-12aα-H-perhydrochrysen kurz als "perhydrochrysen" bezeichnet):

2α-Cyan-2ß-propyl-7α-ethyl-perhydrochrysen
2α-Cyan-2ß-propyl-7α-propyl-perhydrochrysen
2α-Cyan-2ß-propyl-7α-butyl-perhydrochrysen
2α-Cyan-2ß-propyl-7α-pentyl-perhydrochrysen
2α-Cyan-2ß-propyl-7α-heptyl-perhydrochrysen
2α-Cyan-2ß-ethyl-7α-ethyl-perhydrochrysen
2α-Cyan-2ß-ethyl-7α-propyl-perhydrochrysen
2α-Cyan-2ß-ethyl-7α-butyl-perhydrochrysen
2α-Cyan-2ß-ethyl-7α-pentyl-perhydrochrysen
2α-Cyan-2ß-ethyl-7α-heptyl-perhydrochrysen
2α-Cyan-2ß-butyl-7α-ethyl-perhydrochrysen
2α-Cyan-2ß-butyl-7α-propyl-perhydrochrysen
2α-Cyan-2ß-butyl-7α-butyl-perhydrochrysen
2α-Cyan-2ß-butyl-7α-pentyl-perhydrochrysen
2α-Cyan-2ß-butyl-7α-heptyl-perhydrochrysen
2α-Cyan-2ß-pentyl-7α-ethyl-perhydrochrysen
2α-Cyan-2ß-pentyl-7α-propyl-perhydrochrysen

2α-Cyan-2β-pentyl-7α-butyl-perhydrochrysen
2α-Cyan-2β-pentyl-7α-pentyl-perhydrochrysen
2α-Cyan-2β-pentyl-7α-heptyl-perhydrochrysen
2α-Cyan-2β-hexyl-7α-ethyl-perhydrochrysen
2α-Cyan-2β-hexyl-7α-propyl-perhydrochrysen
2α-Cyan-2β-hexyl-7α-butyl-perhydrochrysen
2α-Cyan-2β-hexyl-7α-pentyl-perhydrochrysen
2α-Cyan-2β-hexyl-7α-heptyl-perhydrochrysen
2α-Cyan-2β-heptyl-7α-ethyl-perhydrochrysen
2α-Cyan-2β-heptyl-7α-propyl-perhydrochrysen
2α-Cyan-2β-heptyl-7α-butyl-perhydrochrysen
2α-Cyan-2β-heptyl-7α-pentyl-perhydrochrysen
2α-Cyan-2β-heptyl-7α-heptyl-perhydrochrysen
2α-Cyan-2β-pentyl-7α-methoxy-perhydrochrysen
2α-Cyan-2β-pentyl-7α-ethoxy-perhydrochrysen
2α-Cyan-2β-pentyl-7α-propoxy-perhydrochrysen
2α-Cyan-2β-pentyl-7α-butoxy-perhydrochrysen
2α-Cyan-2β-pentyl-7α-propionyloxy-perhydrochrysen
2α-Cyan-2β-pentyl-7α-butyryloxy-perhydrochrysen
2α-Cyan-2β-pentyl-7α-pentanoyloxy-perhydrochrysen
2α-Cyan-2β-pentyl-7α-2-oxabutyl-perhydrochrysen
2α-Cyan-2β-pentyl-7α-3-oxabutyl-perhydrochrysen

Es folgen Beispiele für erfindungsgemäße Dielektrika mit einem Gehalt an mindestens einer Verbindung der Formel I:

Beispiel A

Man stellt ein Gemisch her aus

10 % 2α-Cyan-2ß-propyl-7α-hexyl-perhydrophenanthren
24 % 2ß-Cyan-2α-heptyl-6ß-propyl-trans-dekalin
22 % 2ß-Cyan-2α-propyl-6ß-pentyl-trans-dekalin
21 % 2ß-Cyan-2α-,6ß-dipentyl-trans-dekalin
13 % 2ß-Cyan-2α-propyl-6ß-heptyl-trans-dekalin und
10 % 4-(trans-4-Pentylcyclohexyl)-4'-(trans-4-propylcyclohexyl)-biphenyl

Beispiel B

In 98 Gewichtsteilen des Gemisches nach Beispiel A löst man 2 Gewichtsteile des blauen Farbstoffs 4,8-Diamino-1,5-dihydroxy-2-p-methoxyphenyl-anthrachinon.

Beispiel C

Man stellt ein Gemisch her aus

30 % 2α-Cyan-2ß-propyl-7α-hexyl-perhydrophenanthren
11 % trans-4-Propylcyclohexancarbonsäure-(p-ethoxyphenylester)
9 % 4-(trans-4-Pentylcyclohexyl)-4'-(trans-4-propylcyclohexyl)-biphenyl
28 % 4-Ethyl-2'-fluor-4'-(trans-4-pentylcyclohexyl)-biphenyl und
22 % trans-1-(p-Ethoxyphenyl)-4-propylcyclohexan.

Beispiel D

Man stellt ein Gemisch her aus

22 % 2ß-Cyan-2α-heptyl-6ß-propyl-trans-dekalin
20 % 2ß-Cyan-2α,6ß-dipentyl-trans-dekalin

18 % trans-4-Pentylcyclohexancarbonsäure-(trans-4-propylcyclohexylester)
0,5 % 2α-Cyan-2β-propyl-7α-hexyl-perhydrophenanthren
9,5 % 4-(trans-4-pentylcyclohexyl)-4'-(trans-4-propylcyclohexyl)-biphenyl und
30 % 4-Ethyl-2'-fluor-4'-(trans-4-pentylcyclohexyl)-biphenyl.

Beispiel E

Man stellt ein Gemisch her aus

17 % 2α-Cyan-2β-propyl-8α-pentyl-perhydrochrysen
43 % trans-4-Propylcyclohexancarbonsäure-(trans-4-propylcyclohexylester)
16 % trans-4-Pentylcyclohexancarbonsäure-(trans-4-propyl-cyclohexylester) und
17 % p-(trans-4-Propylcyclohexyl)-benzoesäure-(4-butyl-2-cyanphenylester).

Merck Patent Gesellschaft
mit beschränkter Haftung

6100 D a r m s t a d t

## Patentansprüche:

1. Carbonitrile der Formel I

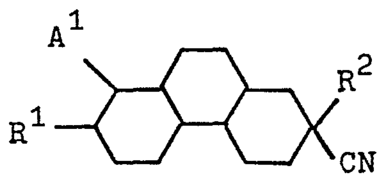

I

worin $R^1$ und $R^2$ jeweils eine Alkylgruppe mit 1 - 12 C-Atomen bedeutet, worin auch eine oder zwei nicht benachbarte $CH_2$-Gruppen durch -O-, -O-CO-, -CO-O- und/oder -CH=CH- ersetzt sein können, und

$A^1$ H, oder 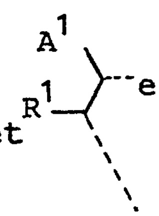 eine Gruppe der Formel 1 bedeutet

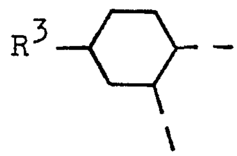

(1)

worin $R^3$ eine der Bedeutungen von $R^1$ hat.

2. Verfahren zur Herstellung von Verbindungen der Formel I nach Anspruch 1, dadurch gekennzeichnet, daß man eine Verbindung der Formel I, worin $R^2$ H bedeutet, mit einer Verbindung der Formel II

$$R^2\text{-}X \qquad II$$

worin X Cl, Br, J, OH oder eine reaktionsfähig veresterte OH-Gruppe bedeutet und
$R^2$ die angegebene Bedeutung hat,

umsetzt.

3. Verwendung der Verbindungen der Formel I nach Anspruch 1 als Komponenten flüssigkristalliner Phasen.

4. Flüssigkristalline Phasen mit mindestens zwei flüssigkristallinen Komponenten, dadurch gekennzeichnet, daß mindestens eine Komponente eine Verbindung der Formel I nach Anspruch 1 ist.

5. Flüssigkristallanzeigeelement, dadurch gekennzeichnet, daß es eine Phase nach Anspruch 4 enthält.

6. Elektrooptisches Anzeigeelement nach Anspruch 5, dadurch gekennzeichnet, daß es als Dielektrikum eine Phase nach Anspruch 4 enthält.

Europäisches Patentamt

**EUROPÄISCHER RECHERCHENBERICHT**

0184068
Nummer der Anmeldung

EP 85 11 4677

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| P,A | EP-A-0 127 816 (MERCK)<br>* Ansprüche * & DE - A - 3 319 781 (Kat. D,A)<br>--- | 1-6 | C 07 C 121/46<br>C 07 C 120/00<br>C 09 K 19/32<br>G 02 F 1/13 |
| A | DE-A-2 747 113 (MERCK)<br>* Ansprüche 1,11,12 *<br>--- | 1,3-6 | |
| A | GB-A-2 082 179 (HOFFMANN-LA ROCHE)<br>* Ansprüche *<br>--- | 1,3-6 | |
| A | US-A-4 434 073 (W. SUCROW et al.)<br>* Ansprüche 1,13-15 * & DE - A - 3 148 448 (Kat. D,A)<br>----- | 1,3-6 | |

| RECHERCHIERTE SACHGEBIETE (Int Cl 4) |
|---|
| C 07 C 121/00<br>C 09 K 19/00 |

Der vorliegende Recherchenbericht wurde für alle Patentansprüche erstellt

| Recherchenort | Abschlußdatum der Recherche | Prüfer |
|---|---|---|
| WIEN | 18-02-1986 | HOFBAUER |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82